# EUROPEAN PATENT APPLICATION

(11) **EP 3 738 595 A1**
(43) Date of publication of application: **18.11.2020**
(21) Application number: 20160070.7
(22) Date of filing: 21.12.2016
(51) Int. Cl.: A61K 31/7068, A61P 35/00

(54) **COMBINATION THERAPY**

(30) Priority: 23.12.2015 WO PCT/GB2015/054158; 03.06.2016 GB 201609770
(62) Divisional of application: 16820005.3
(71) Applicant: Nucana PLC, Edinburgh EH12 9DT (GB)
(72) Inventor: GRIFFITH, Hugh, Edinburgh, EH12 9DT (GB)
(74) Representative: HGF

(57) **Abstract**

This invention relates to a combination of gemcitabine-[phenyl-benzoxy-L-alaninyl)]-phosphate (chemical name: 2'-Deoxy-2',2'-difluoro-D-cytidine-5'-O-[phenyl (benzoxy- L-alaninyl)] phosphate) (NUC-1031) and a platinum-based anticancer agent selected from cisplatin, picoplatin, lipoplatin and triplatin. The combinations are useful in the treatment of cancer and particularly biliary tract and bladder cancer.

## Description

This invention relates to a combination of gemcitabine-[phenyl-benzoxy-L-alaninyl)]-phosphate (chemical name: 2'-Deoxy-2',2'-difluoro-D-cytidine-5'-O-[phenyl (benzoxy- L-alaninyl)] phosphate) (NUC-1031) and a platinum-based anticancer agent selected from cisplatin, picoplatin, lipoplatin and triplatin.

### BACKGROUND

### NUC-1031

Gemcitabine (**1**; marketed as Gemzar®) is an effective nucleoside analogue that is currently approved to treat breast, non-small cell lung, ovarian and pancreatic cancers and widely used to treat a variety of other cancers including bladder, biliary, colorectal and lymphoma.

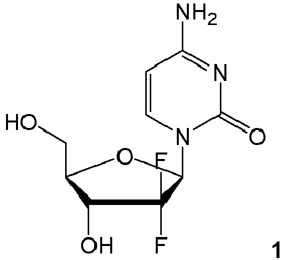

Gemcitabine's clinical utility is limited by a number of inherent and acquired resistance mechanisms. At the cellular level resistance is dependent on three parameters:
(i) the down-regulation of deoxycytidine kinase, necessary for the activation into the phosphorylated moiety; (ii) the reduced expression of nucleoside transporters, in particular, hENT1 required for uptake by cancer cells; and (iii) the up-regulation of catalytic enzymes especially cytidine deaminase that degrades gemcitabine.

WO2005/012327 describes a series of nucleotide analogues for gemcitabine and related nucleoside drug molecules. Among them gemcitabine-[phenyl-benzoxy-L-alaninyl)]-phosphate (NUC-1031; **2**) is identified as a particularly effective compound. These protides avoid many of the inherent and acquired resistance mechanisms which limit the utility of gemcitabine ('Application of ProTide Technology to Gemcitabine: A Successful Approach to Overcome the Key Cancer Resistance Mechanisms Leads to a New Agent (NUC-1031) in Clinical Development'; Slusarczyk et al; J. Med. Chem.; 2014, 57, 1531-1542).

NUC-1031 2 is typically prepared as a mixture of two diastereoisomers, epimeric at the phosphate centre (the S-epimer **3** and the R-epimer **4**), which can be separated and administered as a single epimer.

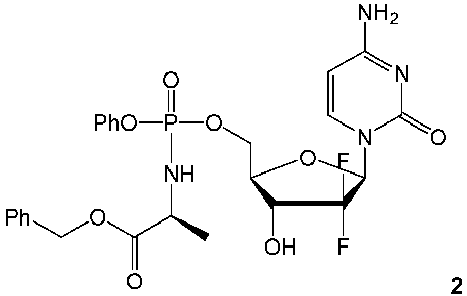

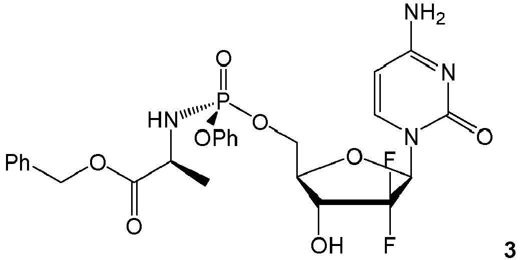

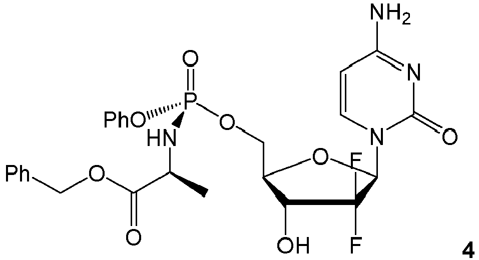

ProGem1 was a first-time-in-human (FTIH), phase I, open label, two stage study to investigate the safety, tolerability, clinical efficacy, pharmacokinetics (PK) and pharmacodynamics (PD) of NUC-1031 given in two parallel dosing schedules in subjects with advanced solid malignancies (EudraCT Number: 2011-005232-26). Subjects had the following tumour types at study entry: colorectal cancer (7 subjects), unknown primary (3), ovarian cancer (12), breast cancer (4), pancreatic cancer (9), cholangiocarcinoma (7), endometrial cancer (3), cervical cancer (2), lung cancer (7), mesothelioma (3), oesophageal cancer (3), cancer of the fallopian tube (1), trophoblast (1), renal cancer (1), gastric cancer (1), anal cancer (1), cancer of the thymus (1) and osteosarcoma (1). The study confirmed NUC-1031's anti-tumour activity in patients with advanced progressive cancers, who have exhausted all standard therapeutic options, many of whom were resistant or refractory to prior nucleoside analogue therapy, including gemcitabine. Of particular note, the pharmacokinetic data showed that NUC-1031 as single agent generates around a10-fold higher peak intracellular concentration (Cₘₐₓ) of the active triphosphate moiety (dFdCTP) than single agent gemcitabine at equimolar dose. Moreover, the intracellular exposure over time or Area Under the Curve (AUC) to dFdCTP, was 27-fold greater for NUC-1031 compared to historical data for gemcitabine from a number of published studies. Finally, the analyses revealed that NUC-1031 releases less than half the levels of the potentially toxic metabolite 2',2'-difluoro-2'-deoxyuridine (dFdU) normally associated with gemcitabine.

### Biliary Tract Cancer

Biliary tract cancers (BTCs) are associated with a high mortality rate (approximately 23 per million population with an incidence of 0.7% malignant tumours in adults, *i.e.* approximately 1200 new cases registered in England and Wales per year. Biliary tract cancers are sub-classified with respect to site of origin as:
- Gallbladder cancer
- Distal bile duct
- Ampullary tumours
- Intra-hepatic cholangiocarcinoma
- Hilar (Klatskin) cholangiocarcinoma

These cancers are more prevalent in patients between 50 and 70 years, with a higher incidence in males in the case of cholangiocarcinoma and ampullary carcinomas, and in females for gallbladder cancers. Although, more than 90% of BTCs are adenocarcinomas, it is possible to find other histological subtypes such as squamous, neuroendocrine tumours, lymphomas or sarcomas. The main aetiological factors for BTC are gallstones, congenital abnormalities of the bile ducts, primary sclerosing cholangitis, chronic liver diseases and hereditary polyposis syndromes.

Surgery offers the only chance of long-term cure; however, due to the aggressive nature of BTC, most patients (>65%) are diagnosed in advanced stages when no surgery is feasible and when palliative chemotherapy is the only treatment available. The prognosis of patients diagnosed with advanced (metastatic or unresectable locally advanced disease) biliary tract cancer is poor. The five-year overall survival for stage III and IV is 10% and 0%, respectively. Nevertheless, first line doublet chemotherapy has shown improvement in overall survival and quality of life compared to single agent therapy.

The most active chemotherapy drugs for the treatment of BTCs are gemcitabine, fluoropyrimidines and platinum agents. The UK NCRN ABC-02 study established cisplatin and gemcitabine as the reference regimen for the first-line treatment of patients with BTC. Results from this randomised phase III study with 410 patients comparing cisplatin/gemcitabine doublet chemotherapy over gemcitabine monotherapy, demonstrated advantage in overall survival (median 11.7 vs. 8.1 months; p<0.001) and in progression-free survival (median 8 vs. 5 months; p<0.001). A very similar magnitude of benefit was seen in a Japanese randomized phase II study using the same treatment regimens (the BT-22 study) where a median survival of 11.2 months was documented with cisplatin/gemcitabine. The robustness of the ABC-02 study given its size and observed survival advantage has established the combination of cisplatin and gemcitabine as the standard of care and has since been widely adopted in the UK and internationally (for example NCCN guidelines in USA).

It is an aim of this invention to provide a combination therapy for treating cancer. It is an aim of certain embodiments of this invention to provide a therapy that is more effective than existing treatments.

Certain embodiments of this invention satisfy some or all of the above aims.

### BRIEF SUMMARY OF THE DISCLOSURE

In accordance with the present invention there is provided gemcitabine-[phenyl-benzoxy-L-alaninyl)]-phosphate, or a pharmaceutically acceptable salt or solvate thereof for use in treating cancer in combination with a platinum-based anticancer agent selected from cisplatin, picoplatin, lipoplatin and triplatin.

The invention also provides gemcitabine-[phenyl-benzoxy-L-alaninyl)]-phosphate, or a pharmaceutically acceptable salt or solvate thereof in combination with a platinum-based anticancer agent selected from cisplatin, picoplatin, lipoplatin and triplatin. The combination will typically be for use in treating cancer.

The invention also provides a platinum-based anticancer agent selected from cisplatin, picoplatin, lipoplatin and triplatin for use in treating cancer in combination with gemcitabine-[phenyl-benzoxy-L-alaninyl)]-phosphate, or a pharmaceutically acceptable salt or solvate thereof.

The invention also provides a method of treating cancer, the method comprising administering to a subject in need thereof a therapeutically effective amount of gemcitabine-[phenyl-benzoxy-L-alaninyl)]-phosphate, or a pharmaceutically acceptable salt or solvate thereof, in combination with a platinum-based anticancer agent selected from cisplatin, picoplatin, lipoplatin and triplatin.

The invention also provides gemcitabine-[phenyl-benzoxy-L-alaninyl)]-phosphate, or a pharmaceutically acceptable salt or solvate thereof, in combination with a platinum-based anticancer agent selected from cisplatin, picoplatin, lipoplatin and triplatin for use in the manufacture of a medicament for treating cancer.

The invention also provides gemcitabine-[phenyl-benzoxy-L-alaninyl)]-phosphate, or a pharmaceutically acceptable salt or solvate thereof, for use in the manufacture of a medicament for treating cancer in combination with a platinum-based anticancer agent selected from cisplatin, picoplatin, lipoplatin and triplatin.

The invention also provides a platinum-based anticancer agent selected from cisplatin, picoplatin, lipoplatin and triplatin for use in the manufacture of a medicament for treating cancer in combination with gemcitabine-[phenyl-benzoxy-L-alaninyl)]-phosphate, or a pharmaceutically acceptable salt or solvate thereof.

The invention also provides a pharmaceutical formulation comprising gemcitabine-[phenyl-benzoxy-L-alaninyl)]-phosphate, or a pharmaceutically acceptable salt or solvate thereof, together with a platinum-based anticancer agent selected from cisplatin, picoplatin, lipoplatin and triplatin, and at least one pharmaceutically acceptable excipient.

The formulation may contain a unit dosage of gemcitabine-[phenyl-benzoxy-L-alaninyl)]-phosphate and a unit dosage of the platinum-based anticancer agent. The unit dosages may be the same but will typically be different.

The invention also provides a two separate formulations to be used together, the formulations being:
a first formulation comprising gemcitabine-[phenyl-benzoxy-L-alaninyl)]-phosphate, or a pharmaceutically acceptable salt or solvate thereof, and at least one pharmaceutically acceptable excipient; and
a second formulation comprising a platinum-based anticancer agent selected from cisplatin, picoplatin, lipoplatin and triplatin and at least one pharmaceutically acceptable excipient.

The formulations may be in the form of a kit. The formulations (i.e. the kit comprising said formulations) will typically be for treating cancer.

The treatments of the present invention are based on the fact that the combination of the two agents (i.e. the gemcitabine-[phenyl-benzoxy-L-alaninyl)]-phosphate and the platinum-based anticancer agent) show greater efficiency when administered in combination than is the case when either is administered alone. The term 'in combination' or 'together' in the context of the present invention refers to the fact that the two agents are both administered to the same patient during the treatment period. The administration may be separate in the sense of being provided in separate doses or may be in the same dose. Administration may take place concurrently or in sequence either immediately one after the other or with a time interval in between the administration of the two agents. The term 'alone' in the context of this discussion thus means administration of only one active agent and no administration of the other agent during the treatment period, even after a time interval.

Combination therapy according to the invention embraces the co-administration or sequential administration of the two active agents in a manner, which enhances the overall therapeutic result relative to the administration of one of the active agents alone during the overall treatment period. The pharmaceutical formulation(s) employed for the purpose may be individual, *i.e.* separate formulations, or presented in a single formulation. The or each formulation may be in a liquid form, either diluted or ready for dilution, or may be in a solid form. Solid forms may be provided for dissolution in a suitable solvent medium. Solid forms may also be presented in concentrated unit dosage form as tablets, capsules losanges etc.

In particular, the present inventors have found that cisplatin sensitises certain cancer cell lines, e.g. bladder cancer cell line HT1376, to NUC-1031 in a strong synergistic effect. Further, the inventors have found that, in vivo, the combination of NUC-1031 and cisplatin leads to an increase in the intra-cellular t_{1/2} of dFdCTP and have shown that the combination can be efficacious in the treatment of biliary tract cancers.

The synergy observed for gemcitabine and platinums has been attributed to an increase by 1.5-fold in levels of dFdCTP (gemcitabine triphosphate) the active metabolite of both gemcitabine and NUC-1031 (van Moorsel et al., British Journal of Cancer, 1999, 80(7), 981-990), which has been described as the result of improved deoxycytidine kinase (dCK) activity. When combined with gemcitabine two platinum-based mechanisms have been suggested to increase dCK-mediated dFdCTP levels. The first cellular mechanism involves ribonucleotide reductase inhibition, the enzyme responsible for deoxycytidine triphosphate (dCTP) synthesis, known to inhibit dCK (Bajetta et al., Annals of Oncology, 2003, 14, 242-247). In the second molecular mechanism the platinum-induced DNA-damage activates the nucleotide excision repair processes, which require deoxyribonucleotides (dNTPs). In turn several enzymes implicated in dNTPs synthesis are up-regulated, including dCK (van Moorsel *et al.,* 1999). NUC-1031 is synthesised as a nucleotide analogue, in the monophosphate form, which bypasses dCK-dependent dFdCTP formation and therefore the synergy observed combining NUC-1031 and cisplatin appears to originate from a different and yet unknown pathway

In certain preferred embodiments, the platinum-based anticancer agent is cisplatin.

The gemcitabine-[phenyl-benzoxy-L-alaninyl)]-phosphate may be a mixture of phosphate diastereoisomers or it may be the (S)-epimer or as the (*R*)-epimer in substantially diastereomerically pure form. 'Substantially diastereomerically pure' is defined for the purposes of this invention as a diastereomeric purity of greater than about 90%. If present as a substantially diastereoisomerically pure form, the gemcitabine-[phenyl-benzoxy-L-alaninyl)]-phosphate may have a diastereoisomeric purity of greater than 95%, 98%, 99%, or even 99.5%.

The cancer may be a solid tumour cancer. The cancer may be a cancer selected from: pancreatic cancer, breast cancer, ovarian cancer, bladder cancer, colorectal cancer, lung cancer, biliary tract cancer (*e.g.* a cancer selected from gallbladder cancer, distal bile duct cancer, ampullary cancer, hilar cholangiocarcinoma and intra-hepatic cholangiocarcinoma), prostate cancer, renal cancer, lymphoma, leukemia, cervical cancer, thymic cancer, a cancer of an unknown primary origin, oesophageal cancer, mesothelioma, adrenal cancer, cancer of the uterus, cancer of the fallopian tube, endometrial cancer, testicular cancer, head and neck cancer, cancer of the central nervous system and germ cell tumours.

In certain preferred embodiments, the cancer is selected from bladder cancer, ovarian cancer, non-small cell lung cancer and biliary tract cancer (*e.g.* a cancer selected from gallbladder cancer, distal bile duct cancer, ampullary cancer, hilar cholangiocarcinoma and intra-hepatic cholangiocarcinoma). In certain preferred embodiments, the cancer is selected from bladder cancer, ovarian cancer and biliary tract cancer (*e.g.* a cancer selected from gallbladder cancer, distal bile duct cancer, ampullary cancer, hilar cholangiocarcinoma and intra-hepatic cholangiocarcinoma). In certain preferred embodiments, the cancer is a biliary tract cancer. In other preferred embodiments, the cancer is a bladder cancer. Combinations in which the platinum-based anticancer agent is cisplatin are particularly preferred for treating these particular cancers. In certain preferred embodiments, the cancer is selected from ovarian cancer, non-small cell lung cancer and biliary tract cancer (e.g. a cancer selected from gallbladder cancer, distal bile duct cancer, ampullary cancer, hilar cholangiocarcinoma and intra-hepatic cholangiocarcinoma) and the platinum-based anticancer agent is cisplatin. In certain preferred embodiments, the cancer is selected from ovarian cancer and biliary tract cancer (*e.g.* a cancer selected from gallbladder cancer, distal bile duct cancer, ampullary cancer, hilar cholangiocarcinoma and intra-hepatic cholangiocarcinoma) and the platinum-based anticancer agent is cisplatin. Thus, it may be that the cancer is biliary tract cancer and the platinum-based anticancer agent is cisplatin. Likewise, it may be that the cancer is bladder cancer and the platinum-based anticancer agent is cisplatin.

The cancer may be previously untreated with chemotherapy. Alternatively, the cancer (*e.g.* the biliary tract or bladder cancer) may be relapsed. Thus, the cancer may have recurred or progressed after one or more prior courses of chemotherapy (which may or may not have included treatment with an agent selected from cisplatin, gemcitabine or gemcitabine-[phenyl-benzoxy-L-alaninyl)]-phosphate. The cancer (*e.g.* the biliary tract or bladder cancer) may be refractory, resistant or partially resistant to the platinum-based anticancer agent *(e.g.* cisplatin). Alternatively, the cancer *(e.g.* the biliary tract or bladder cancer) may be sensitive to the platinum-based anticancer agent (*e.g.* cisplatin). The cancer (*e.g.* the biliary tract or bladder cancer) may be metastatic.

A solvate will typically be a hydrate. Thus, the gemcitabine-[phenyl-benzoxy-L-alaninyl)]-phosphate may be in the form of a salt or hydrate, or a solvate (e.g. hydrate) of a salt. It may be that the gemcitabine-[phenyl-benzoxy-L-alaninyl)]-phosphate is not in the form of a salt and it may be that it is not in the form of a solvate or hydrate. Preferably, the gemcitabine-[phenyl-benzoxy-L-alaninyl)]-phosphate is in the form of the free base.

It may be that administration of the combination provides an intra-cellular t_{1/2} of dFdCTP of more than 10 hours. It may be that administration of the combination provides an intra-cellular t_{1/2} of dFdCTP of more than 15 hours. It may be that administration of the combination provides an intra-cellular t_{1/2} of dFdCTP of more than 18 hours. It may be that administration of the combination provides an intra-cellular t_{1/2} of dFdCTP of more than 20 hours.

The gemcitabine-[phenyl-benzoxy-L-alaninyl)]-phosphate and the platinum-based anticancer agent may be administered simultaneously or they may be administered sequentially. Where they are administered simultaneously, they may be administered in a single formulation or they may be administered in separate formulations. Where they are administered sequentially, they may be administered on the same day or they may be administered on separate days during the treatment period. It may be that on certain days during the treatment period, the gemcitabine-[phenyl-benzoxy-L-alaninyl)]-phosphate and the platinum-based anticancer agent are administered simultaneously or on the same day and on certain other days in the treatment program a single one of the agents is administered.

### NUC-1031 formulations

The gemcitabine-[phenyl-benzoxy-L-alaninyl)]-phosphate may be administered parenterally, e.g. intravenously, subcutaneously or intramuscularly. Preferably, the gemcitabine-[phenyl-benzoxy-L-alaninyl)]-phosphate is administered intravenously.

The gemcitabine-[phenyl-benzoxy-L-alaninyl)]-phosphate may be administered parenterally as an aqueous formulation which optionally also comprises a polar organic solvent, *e.g.* DMA. In the case of parenteral (*e.g.* intravenous) administration, the formulation preferably also comprises a polar aprotic organic solvent, *e.g.* DMA.

The gemcitabine-[phenyl-benzoxy-L-alaninyl)]-phosphate may be comprised in a formulation. The formulation may be for dilution by a predetermined amount shortly before administration, *i.e.* up to 48 hours (*e.g.* up to 24, 12 or 2 hours) before administration.

The formulation may also comprise one or more pharmaceutically acceptable solubilizers, e.g. a pharmaceutically acceptable non-ionic solubilizers. Solubilizers may also be called surfactants or emulsifiers. Illustrative solubilizers include polyethoxylated fatty acids and fatty acid esters and mixtures thereof. Suitable solubilizers may be or comprise polyethoxylated castor oil (*e.g.* that sold under the trade name Kolliphor® ELP); or may be or comprise polyethoxylated hydroxy-stearic acid (*e.g.* that sold under the trade names Solutol® or Kolliphor® HS15); or may be or comprise polyethoxylated (*e.g.* polyoxyethylene (20)) sorbitan monooleate, (e.g. that sold under the trade name Tween® 80).

In certain preferred embodiments, the formulation comprises more than one pharmaceutically acceptable solubilizer.

The formulation may also comprise an aqueous vehicle. The formulation may be ready to administer, in which case it will typically comprise an aqueous vehicle.

While gemcitabine-[phenyl-benzoxy-L-alaninyl)]-phosphate is preferably formulated for parenteral administration *e.g.* for intravenous, subcutaneous or intramuscular administration, in certain embodiments of the invention it may be administered orally. Preferably, the formulation is for intravenous administration. The administration may be through a Central Venous Administration Device (CVAD) or it may be through a peripheral vein.

The total dose of gemcitabine-[phenyl-benzoxy-L-alaninyl)]-phosphate in a formulation suitable for administration will typically be from 250 mg to 3 g, *e.g.* from 1 g to 2 g, *e.g.* about 1.5 g.

### Stock solution formulations

It may be that the polar aprotic solvent (e.g. DMA) represents 30% or more by volume of the formulation. Thus, it may be that the polar aprotic solvent (e.g. DMA) represents 50% or more, e.g. 60% or more by volume of the formulation. The polar aprotic solvent (e.g. DMA) may represent 95% or less by volume of the formulation, e.g. 90% or less. The formulation may also comprise an aqueous vehicle (e.g. saline). The aqueous vehicle may be present in 50% or less by volume of the formulation, e.g. 30% or less by volume of the formulation. Typically the aqueous vehicle (e.g. saline) will represent 5% or more, e.g. 10% or more, by volume of the formulation.

It may be that the concentration of gemcitabine-[phenyl-benzoxy-L-alaninyl)]-phosphate in the formulation solvent(s) is 500 mg or less per mL. It may be that the concentration 100 mg or more per mL. Preferably, the concentration is from 200 mg to 300 mg, e.g. from 225 mg to 275 mg, e.g. about 250 mg, per mL.

Certain preferred formulations comprise:
from 30 % to 95% by volume DMA;
from 5% to 50% by volume aqueous vehicle; and
from 100 mg to 400 mg (e.g. from 100 mg to 300 mg) per mL gemcitabine-[phenyl-benzoxy-L-alaninyl)]-phosphate.

More preferred formulations comprise:
from 70 % to 90% by volume DMA;
from 10% to 30% by volume aqueous vehicle (e.g. saline); and
from 200 mg to 300 mg per mL gemcitabine-[phenyl-benzoxy-L-alaninyl)]-phosphate.

The formulations described in the previous four paragraphs, in which the polar aprotic solvent (e.g. DMA) is present as a major component may be for administering (e.g. by infusion or injection) the formulation without it being diluted prior to said administration. They may, for example, be for administration through a CVAD. When administered via a CVAD, the formulation is typically not diluted.

Alternatively, these formulations may be stock solutions which are diluted prior to use to form a formulation suitable for administration, e.g. through a peripheral vein.

### Surfactant solution formulations

It may be that the polar aprotic solvent (e.g. DMA) represents 10% or more, e.g. 20% or more by volume of the formulation. Thus, it may be that the polar aprotic solvent (e.g. DMA) represents 80% or less, e.g. 70% or less by volume of the formulation. The polar aprotic solvent (e.g. DMA) may represent 55% or less by volume of the formulation. The formulation may also comprise one or more solubilizers (e.g. one or more polyethoxylated fatty acids). The one or more solubilizers may represent 70% or less by volume of the formulation, e.g. 60% or less by volume of the formulation. Typically the one or more solubilizers will represent 20% or more, e.g. 35%, by volume of the formulation. The formulation may also comprise an aqueous vehicle, e.g. in an amount from 1% to 15% by volume or from 5% to 12% by volume.

It may be that the concentration of gemcitabine-[phenyl-benzoxy-L-alaninyl)]-phosphate in the formulation solvent(s) is 200 mg or less per mL, e.g. 150mg or less or 130 mg or less. It may be that the concentration is 40 mg or more per mL, e.g. 60 mg or more. Preferably, the concentration is from 70 mg to 120 mg per mL, e.g. about 100 mg per mL.

Certain preferred formulations comprise:
from 20 % to 70% by volume DMA;
from 20% to 70% by volume solubilizer or solubilizers; and
from 50 mg to 150 mg per mL gemcitabine-[phenyl-benzoxy-L-alaninyl)]-phosphate. The formulation may also comprise an aqueous vehicle, e.g. in an amount from 1% to 15% by volume.

Certain particularly preferred formulations comprise:
from 30 % to 60% by volume DMA;
from 10% to 35% by volume a first solubilizer;
from 10% to 35% by volume a second solubilizer;
from 2% to 15% by volume an aqueous vehicle; and
from 50 mg to 150 mg per mL gemcitabine-[phenyl-benzoxy-L-alaninyl)]-phosphate. The first solubilizer may be a polyethoxylated castor oils (e.g. that sold under the trade name Kolliphor® ELP).The second solubilizer may be a polyethoxylated sorbitan monooleate (e.g. that sold under the trade name Tween® 80).

The formulation may comprise:
from 35 % to 50% by volume DMA;
from 15% to 30% by volume the first solubilizer;
from 15% to 30% by volume the second solubilizer;
from 5% to 12% by volume an aqueous vehicle; and
from 50 mg to 150 mg per mL gemcitabine-[phenyl-benzoxy-L-alaninyl)]-phosphate.

The surfactant solutions formulations described in the previous five paragraphs, in which the polar aprotic solvent (e.g. DMA) is present as a major component are typically diluted with an aqueous vehicle prior to administration. They are typically prepared from the stock solutions mentioned above before being further diluted ready for administration. Once diluted, they may be administered through a peripheral vein.

These formulations may be formed by diluting a stock solution formulation that does not contain any solubilizers with a solution which does contain solubilizers.

### Infusion solution formulations

It may be that the polar aprotic solvent (e.g. DMA) represents 0.1% or more, e.g. 0.5% or more or 1% or more by volume of the formulation. Thus, it may be that DMA represents 12% or less, e.g. 10% or less or 8% or less by volume of the formulation. The formulation may also comprise an aqueous vehicle (e.g. saline or WFI). The aqueous vehicle may be present in 99.5% or less by volume of the formulation, e.g. 99% or 98% or less by volume of the formulation. Typically the aqueous vehicle will represent 80% or more, e.g. 95% or more, by volume of the formulation. The formulation may also comprise one or more solubilizers (e.g. one or more polyethoxylated fatty acids). The one or more solubilizers may present in 12% or less by volume of the formulation, e.g. 10% or less or 8% or less by volume of the formulation. Typically the one or more solubilizers will be present in 0.1% or more, e.g. 0.5% or more or 1% or more, by volume of the formulation.

It may be that the concentration of gemcitabine-[phenyl-benzoxy-L-alaninyl)]-phosphate in the formulation solvent(s) is 15.0 mg or less per mL or 12.0 mg or less per mL, e.g. 10.0 mg or less or 8 mg or less per mL. It may be that the concentration is 1.0 mg or more per mL, e.g. 2.0 mg or more. Preferably, the concentration is from 2.5 mg to 12 mg per mL, e.g. from 3 mg to 11 mg per mL.

Certain preferred formulations comprise:
from 0.1 % to 10% by volume DMA;
from 0.1% to 10% by volume solubilizer or solubilizers;
from 85% to 99% by volume aqueous vehicle; and
from 2.0 mg to 12.0 mg per mL gemcitabine-[phenyl-benzoxy-L-alaninyl)]-phosphate.

Certain particularly preferred formulations comprise:
from 1 % to 8% by volume DMA;
from 0.5 % to 4% by volume a first solubilizer;
from 0.5 % to 4% by volume a second solubilizer;
from 85% to 99% by volume aqueous vehicle; and
from 2.0 mg to 12.0 mg per mL gemcitabine-[phenyl-benzoxy-L-alaninyl)]-phosphate. The first solubilizer may be a polyethoxylated castor oil (e.g. that sold under the trade name Kolliphor® ELP). The second solubilizer may be a polyethoxylated sorbitan monooleate (e.g. that sold under the trade name Tween® 80).

The infusion solution formulations described in the previous four paragraphs, in which the polar aprotic solvent (e.g. DMA) is present as a minor component, will typically have been prepared by diluting a concentrated solution of gemcitabine-[phenyl-benzoxy-L-alaninyl)]-phosphate with the aqueous vehicle up to 48 hours prior to administration. Said concentrated solution may be either a solution of gemcitabine-[phenyl-benzoxy-L-alaninyl)]-phosphate in a polar aprotic solvent (see under the heading 'stock solution formulation' above) a solution of gemcitabine-[phenyl-benzoxy-L-alaninyl)]-phosphate in mixture of a polar aprotic solvent and a solubilizer (see under the heading 'surfactant solution formulation' above). These formulations in which the polar aprotic solvent (e.g. DMA) is present as a minor component may be administered through a peripheral vein. The low concentrations of the polar aprotic solvent (e.g. DMA) in said formulations mean that they tend not to cause pain upon peripheral administration.

### Kits

The invention provides a kit for treating cancer, the kit comprising:
a first formulation comprising gemcitabine-[phenyl-benzoxy-L-alaninyl)]-phosphate, or a pharmaceutically acceptable salt or solvate thereof, and at least one pharmaceutically acceptable excipient; and
a second formulation comprising a platinum-based anticancer agent and at least one pharmaceutically acceptable excipient.

In certain particular embodiments, the kit may comprise:
a first formulation comprising:
   from 30 % to 95% by volume DMA;
   from 5% to 50% by volume aqueous vehicle; and
   from 100 mg to 400 mg (e.g. from 100 mg to 300 mg) per mL gemcitabine-[phenyl-benzoxy-L-alaninyl)]-phosphate;
a second formulation comprising a platinum-based anticancer agent and at least one pharmaceutically acceptable excipient; and
a third formulation comprising:
   from 30 % to 95% by volume DMA;
   from 5% to 50% by volume aqueous vehicle.

The third formulation will typically not comprise an active. Thus, it will typically comprise neither gemcitabine-[phenyl-benzoxy-L-alaninyl)]-phosphate nor a platinum-based anticancer agent. The third formulation may be provided in two separate vessels or in a single vessel.

The kit mentioned in the previous two paragraphs is useful where the gemcitabine-[phenyl-benzoxy-L-alaninyl)]-phosphate is administered intravenously via a CVAD. The CVAD is flushed with the third formulation prior to administration of the first formulation. This mitigates the risk of precipitation of gemcitabine-[phenyl-benzoxy-L-alaninyl)]-phosphate in or at the entrance to the intravenous administration apparatus, *i.e.* the CVAD, by avoiding the direct contact of the active formulation with aqueous media (e.g. a saline flushing solution). The CVAD may also be flushed with the third formulation after administration of the first formulation. This further prevents precipitation.

In certain particular embodiments, the kit may comprise:
a first formulation comprising:
   from 30 % to 95% by volume DMA;
   from 5% to 50% by volume aqueous vehicle; and
   from 100 mg to 400 mg (e.g. from 100 mg to 300 mg) per mL gemcitabine-[phenyl-benzoxy-L-alaninyl)]-phosphate;
a second formulation comprising a platinum-based anticancer agent and at least one pharmaceutically acceptable excipient; and
a third formulation comprising:
   from 10 % to 50% by volume DMA;
   from 20% to 60% by volume a first solubilizer;
   from 20% to 60% by volume a second solubilizer.

Typically the third formulation will not comprise any active. Thus, it will typically comprise neither gemcitabine-[phenyl-benzoxy-L-alaninyl)]-phosphate nor a platinum-based anticancer agent.

The kit mentioned in the previous two paragraphs is useful where the gemcitabine-[phenyl-benzoxy-L-alaninyl)]-phosphate is administered intravenously via a peripheral vein. The first formulation is diluted with the third formulation up to 48h, e.g. up to 24h before administration to form a fourth formulation. The fourth formulation is further diluted with an aqueous vehicle before administration to the desired concentration to form the formulation, which is used administered by infusion or injection to the patient. In order to achieve formulations for peripheral administration which are stable with respect to precipitation of gemcitabine-[phenyl-benzoxy-L-alaninyl)]-phosphate, it is typically desirable to include solubilizers. However, the gemcitabine-[phenyl-benzoxy-L-alaninyl)]-phosphate can be prone to degradation in the presence of such solubilizers. Thus, a two stage dilution method is, in certain embodiments of the invention, the preferable means by which formulations of gemcitabine-[phenyl-benzoxy-L-alaninyl)]-phosphate for peripheral administration are achieved.

### Illustrative methodology for administration of gemcitabine-[phenyl-benzoxy-L-alaninyl)]-phosphate

An illustrative methodology for administration of gemcitabine-[phenyl-benzoxy-L-alaninyl)]-phosphate is as follows:
A 250 mg/mL solution of the gemcitabine-[phenyl-benzoxy-L-alaninyl)]-phosphate (the S-epimer, the R epimer or a mixture thereof) is formed in an 80:20 (by volume) mixture of DMA and 0.9% saline. This stock solution formulation is typically sufficiently stable for long term storage and transport of protides. This stock solution formulation can be administered to patients intravenously via a CVAD (e.g. a Hickman line, PICC line, Portacath), e.g. at a rate of 20 ml/hour. The intravenous administration apparatus will typically be flushed with an 80:20 (by volume) mixture of DMA and 0.9% saline (the Flushing Solution mentioned in Example 4 below) both before and after administration of the formulation comprising the gemcitabine-[phenyl-benzoxy-L-alaninyl)]-phosphate. This helps mitigate the risk of any potential precipitation of gemcitabine-[phenyl-benzoxy-L-alaninyl)]-phosphate in the intravenous administration apparatus on contact with the saline flush. Alternatively, where intravenous administration into a peripheral vein is the preferred method of administration the stock solution formulation is then diluted to 100 mg/mL with a diluent solution which is 20%:40%:40% mixture of DMA:Tween® 80:Kolliphor® ELP (e.g. 6.7 mL of 250 mg/ml gemcitabine-[phenyl-benzoxy-L-alaninyl)]-phosphate in 80:20 DMA:0.9% saline is added to 10 mL of the DMA:Tween®80:Kolliphor® ELP diluent solution). The resultant (surfactant solution) formulation is typically stable for up to 5 days. The infusion solution formulation is then prepared by diluting this surfactant solution formulation to the desired concentration with 0.9% saline.

The gemcitabine-[phenyl-benzoxy-L-alaninyl)]-phosphate administered in the ABC-08 study described in the examples was carried out using this administration methodology, with the S epimer of gemcitabine-[phenyl-benzoxy-L-alaninyl)]-phosphate being administered via a CVAD.

### Formulations of the platinum-based anticancer agent

The platinum-based anticancer agent may be administered parenterally, e.g. intravenously, intraperitoneally, subcutaneously or intramuscularly. Preferably, the platinum-based anticancer agent is administered intravenously.

The platinum-based anticancer agent will typically be administered as an aqueous solution, e.g. as a sterile 1 mg/mL aqueous solution. The aqueous solution will typically be a saline solution (e.g. 0.9% saline solution). The aqueous solution may also comprise mannitol (e.g. at 10 mg/mL).

Where the platinum-based anticancer (e.g. cisplatin) agent is administered at a dose less than 50 mg/mL it is typically administered as an infusion from a 100-250mL bag over 15-60 minutes. Where the platinum-based anticancer (e.g. cisplatin) agent is administered at a dose greater than or equal to 50 mg/mL, it is typically administered as an infusion from a 250 to 500 mL bag over 15 to 60 minutes.

Further information on the administration of cisplatin is available, for example, on the US FDA approved label for Platinol®.

### Dosage Regimens

It may be that the NUC-1031 is administered twice in a 21 day cycle. It may be that the platinum-based anticancer agent (e.g. cisplatin) is administered twice in the 21 day cycle. In a preferred dosage regimen NUC-1031 is administered on day 1 and day 8 of a 21 day cycle. It may also be that the platinum-based anticancer agent (*e.g.* cisplatin) is administered on day 1 and day 8 of the 21 day cycle. It may be that NUC-1031 and the platinum-based anticancer agent (*e.g.* cisplatin) are administered simultaneously on day 1 and day 8 of a 21 day cycle.

The dose of NUC-1031 administered at each administration event is preferably in the range from 250 mg/m² to 1250 mg/m². The dose of NUC-1031 administered at each administration event may be in the range from 300 mg/m² to 1000 mg/m². The dose of NUC-1031 administered at each administration event may be in the range from 400 mg/m² to 900 mg/m², *e.g.* from 600 mg/m² to 800 mg/m², or from 300 to 750 mg/m². The dose of NUC-1031 administered at each administration event may be about 750 mg/m².

The dose of the platinum-based anticancer agent (e.g. cisplatin) administered at each administration event may be from 10 mg/m² to 200 mg/m². The dose of the platinum-based anticancer agent (*e.g.* cisplatin) administered at each administration event may be from 20 mg/m² to 100 mg/m². The dose of the platinum-based anticancer agent (*e.g.* cisplatin) administered at each administration event may be from 20 mg/m² to 60 mg/m². The dose of the platinum-based anticancer agent (e.g. cisplatin) administered at each administration event may be from 30 mg/m² to 90 mg/m².

It may be that the dose of NUC-1031, or the dose of the platinum-based anticancer agent (*e.g.* cisplatin), or the dose of both of the compounds, remains substantially the same in each treatment cycle. For example, a dose of NUC-1031 of about 750 mg/m² per administration event, and a dose of cisplatin of about 50 mg/m² may be used in multiple treatment cycles.

Alternatively, it may be that the dose of NUC-1031, or the dose of the platinum-based anticancer agent (*e.g.* cisplatin), or the dose of both of the compounds, decreases from the first treatment cycle to the second (or subsequent) treatment cycle. For example, the dose of NUC-1031 administered at each administration event may decrease from about 750 mg/m², in a first treatment cycle, to about 625 mg/m² in a second (or subsequent) treatment cycle. The dose of the platinum-based anticancer agent (*e.g.* cisplatin) may decrease from about 90 mg/m² in a first cycle of treatment, to about 60 mg/m², or to about 50 mg/m² in a second (or subsequent) treatment cycle.

Suitable treatment regimens may make use of decreases (as set out in the preceding paragraph) in both doses of NUC-1031 and doses of the platinum-based anticancer agent (*e.g.* cisplatin) from a first treatment cycle to a second (or subsequent) treatment cycle. For example, the dose of NUC-1031 administered at each administration event may decrease from about 750 mg/m², in a first treatment cycle, to about 625 mg/m² in a second (or subsequent) treatment cycle, and the dose of the platinum-based anticancer agent (*e.g.* cisplatin) may decrease from about 90 mg/m² in a first cycle of treatment, to about 60 mg/m², or to about 50 mg/m² in a second (or subsequent) treatment cycle.

In the event that the dose of NUC-1031 decreases from a first to a second, or subsequent, treatment cycle (such as from about 750 mg/m² per administration incident, to about 625 mg/m² per administration incident), the dose of the platinum-based anticancer agent (*e.g.* cisplatin) may remain the same between the first and second, or subsequent, treatment cycles (for example, about 50 mg/m² in each cycle).

In the event that the dose of NUC-1031 remains constant from a first to a second, or subsequent, treatment cycle (such as about 625 mg/m² per administration incident), the dose of the platinum-based anticancer agent (*e.g.* cisplatin) may decrease between the first and second, or subsequent, treatment cycles (for example, from 90 mg/m² in a first cycle of treatment, to about 60 mg/m², or to about 50 mg/m² in a second, or subsequent, treatment cycle).

It is expected that the above mentioned dosage regimen provide a balance in which the toxicity of each of the components of the combination is at an acceptable level yet a therapeutic benefit from the combination is still observed.

It may be that the above mentioned dosage regimen provides an improved survival rate in patients. It may be that it provides a stable disease in greater than 50% of patients. It may be that it provides one or more of the above benefits with an acceptable level of side-effects. It may be that the dosage is such that the AUC of dFdCTP is higher for the combination than for NUC-1031 administered as a single agent. It may be that the dosage is such that the ratio of AUC to Cₘₐₓ of dFdCTP is higher for the combination than for NUC-1031 administered as a single agent.

### BRIEF DESCRIPTION OF THE DRAWINGS

Embodiments of the invention are further described hereinafter with reference to the accompanying drawings, in which:
Figure 1 shows the chromatograph for separation of compounds **3** and **4** by HPLC using a Chiralpak AD column and a n-heptane/IPA gradient solvent system.
Figure 2 shows a synergy effect shown using the curve shift method for cisplatin/NUC-1031 in the bladder cancer cell line HT1376

### DETAILED DESCRIPTION

'Simultaneous' is intended to mean "substantially simultaneous" e.g. less than 30 mins apart. 'Sequential' means administration more than 30 mins apart.

Throughout this specification, the term S-epimer or S-diastereoisomer refers to gemcitabine-[phenyl-benzoxy-L-alaninyl)]-(S)-phosphate. Likewise, throughout this specification, the term R-epimer or R-diastereoisomer refers to gemcitabine-[phenyl-benzoxy-L-alaninyl)]-(R)-phosphate.

The compounds of the invention may be obtained, stored and/or administered in the form of a pharmaceutically acceptable salt. Suitable pharmaceutically acceptable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulphonic, toluenesulphonic, benzenesulphonic, salicylic, sulphanilic, aspartic, glutamic, edetic, stearic, palmitic, oleic, lauric, pantothenic, tannic, ascorbic and valeric acids. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulfate, hemioxalate and hemicalcium salts. In certain embodiments, particularly those that apply to the s-epimer, the compound is in the form of a HCI salt or a hemioxalate salt.

Compounds of the invention may exist in a single crystal form or in a mixture of crystal forms or they may be amorphous. Thus, compounds of the invention intended for pharmaceutical use may be administered as crystalline or amorphous products. They may be obtained, for example, as solid plugs, powders, or films by methods such as precipitation, crystallization, freeze drying, or spray drying, or evaporative drying. Microwave or radio frequency drying may be used for this purpose.

For the above-mentioned compounds of the invention the dosage administered will, of course, vary with the compound employed, the mode of administration, the treatment desired and the disorder indicated. For example, if the compound of the invention is administered parenterally, then the dosage of the compound of the invention may be in the range from 0.1 to 5 g/m², e.g. from 0.5 to 2 g/m². The size of the dose for therapeutic purposes of compounds of the invention will naturally vary according to the nature and severity of the conditions, the age and sex of the animal or patient and the route of administration, according to well known principles of medicine.

Dosage levels, dose frequency, and treatment durations of compounds of the invention are expected to differ depending on the formulation and clinical indication, age, and co-morbid medical conditions of the patient.

A compound of the invention, or pharmaceutically acceptable salt thereof, may be used on their own but will generally be administered in the form of a pharmaceutical composition in which the compounds of the invention, or pharmaceutically acceptable salt thereof, is in association with a pharmaceutically acceptable adjuvant, diluent or carrier. Conventional procedures for the selection and preparation of suitable pharmaceutical formulations are described in, for example, "Pharmaceuticals - The Science of Dosage Form Designs", M. E. Aulton, Churchill Livingstone, 1988.

Depending on the mode of administration of the compounds of the invention, the pharmaceutical composition which is used to administer the compounds of the invention will preferably comprise from 0.05 to 99 %w (per cent by weight) compounds of the invention, more preferably from 0.05 to 80 %w compounds of the invention, still more preferably from 0.10 to 70 %w compounds of the invention, and even more preferably from 0.10 to 50 %w compounds of the invention, all percentages by weight being based on total composition.

For oral administration the compounds of the invention may be admixed with an adjuvant or a carrier, for example, lactose, saccharose, sorbitol, mannitol; a starch, for example, potato starch, corn starch or amylopectin; a cellulose derivative; a binder, for example, gelatine or polyvinylpyrrolidone; and/or a lubricant, for example, magnesium stearate, calcium stearate, polyethylene glycol, a wax, paraffin, and the like, and then compressed into tablets. If coated tablets are required, the cores, prepared as described above, may be coated with a concentrated sugar solution, which may contain, for example, gum arabic, gelatine, talcum and titanium dioxide. Alternatively, the tablet may be coated with a suitable polymer dissolved in a readily volatile organic solvent.

For the preparation of soft gelatine capsules, the compounds of the invention may be admixed with, for example, a vegetable oil or polyethylene glycol. Hard gelatine capsules may contain granules of the compound using either the above-mentioned excipients for tablets. Also liquid or semisolid formulations of the compound of the invention may be filled into hard gelatine capsules.

Liquid preparations for oral application may be in the form of syrups or suspensions, for example, solutions containing the compound of the invention, the balance being sugar and a mixture of ethanol, water, glycerol and propylene glycol. Optionally such liquid preparations may contain colouring agents, flavouring agents, sweetening agents (such as saccharine), preservative agents and/or carboxymethylcellulose as a thickening agent or other excipients known to those skilled in art.

For parenteral (e.g. intravenous) administration the compounds may be administered as a sterile aqueous or oily solution. The compounds of the invention are very lipophillic. Aqueous formulations will typically, therefore, also contain a pharmaceutically acceptable polar organic solvent.

The size of the dose for therapeutic purposes of compounds of the invention will naturally vary according to the nature and severity of the conditions, the age and sex of the animal or patient and the route of administration, according to well known principles of medicine.

Dosage levels, dose frequency, and treatment durations of compounds of the invention are expected to differ depending on the formulation and clinical indication, age, and co-morbid medical conditions of the patient.

The present invention also includes all pharmaceutically acceptable isotopically-labelled forms of compounds **2, 3** or **4** wherein one or more atoms are replaced by atoms having the same atomic number, but an atomic mass or mass number different from the atomic mass or mass number of the predominant isotope usually found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds, for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds can generally be prepared by conventional techniques known to those skilled in the art or by processes analogous to those described using an appropriate isotopically-labelled reagent in place of the non-labelled reagent previously employed.

The method of treatment or the compound for use in the treatment of cancer may involve, in addition to the gemcitabine-[phenyl-benzoxy-L-alaninyl)]-phosphate and the platinum-base anticancer compound, conventional surgery or radiotherapy or chemotherapy. Such chemotherapy may include the administration of one or more other active agents.

Thus, each or any one of the pharmaceutical formulations may comprise another active agent.

The one or more other active agents may be one or more of the following categories of anti-tumour agents:
(i) antiproliferative/antineoplastic drugs and combinations thereof, such as alkylating agents (for example cyclophosphamide, nitrogen mustard, bendamustin, melphalan, chlorambucil, busulphan, temozolamide and nitrosoureas); antimetabolites (for example gemcitabine and antifolates such as fluoropyrimidines like 5-fluorouracil and tegafur, raltitrexed, methotrexate, pemetrexed, cytosine arabinoside, and hydroxyurea);; antimitotic agents (for example vinca alkaloids like vincristine, vinblastine, vindesine and vinorelbine and taxoids like taxol and taxotere and polokinase inhibitors); proteasome inhibitors, for example carfilzomib and bortezomib; interferon therapy; and topoisomerase inhibitors (for example epipodophyllotoxins like etoposide and teniposide, amsacrine, topotecan, mitoxantrone and camptothecin);
(ii) cytostatic agents such as antiestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride;
(iii) anti-invasion agents, for example dasatinib and bosutinib (SKI-606), and metalloproteinase inhibitors, inhibitors of urokinase plasminogen activator receptor function or antibodies to Heparanase;
(iv) inhibitors of growth factor function: for example such inhibitors include growth factor antibodies and growth factor receptor antibodies, for example the anti-erbB2 antibody trastuzumab [Herceptin™], the anti-EGFR antibody panitumumab, the anti-erbB1 antibody cetuximab, tyrosine kinase inhibitors, for example inhibitors of the epidermal growth factor family (for example EGFR family tyrosine kinase inhibitors such as gefitinib, erlotinib and 6-acrylamido-*N*-(3-chloro-4-fluorophenyl)-7-(3-morpholinopropoxy)-quinazolin-4-amine (Cl 1033), erbB2 tyrosine kinase inhibitors such as lapatinib); inhibitors of the hepatocyte growth factor family; inhibitors of the insulin growth factor family; modulators of protein regulators of cell apoptosis (for example Bcl-2 inhibitors); inhibitors of the platelet-derived growth factor family such as imatinib and/or nilotinib (AMN107); inhibitors of serine/threonine kinases (for example Ras/Raf signalling inhibitors such as farnesyl transferase inhibitors, for example sorafenib , tipifarnib and lonafarnib), inhibitors of cell signalling through MEK and/or AKT kinases, c-kit inhibitors, abl kinase inhibitors, Pl3 kinase inhibitors, Plt3 kinase inhibitors, CSF-1 R kinase inhibitors, IGF receptor, kinase inhibitors; aurora kinase inhibitors and cyclin dependent kinase inhibitors such as CDK2 and/or CDK4 inhibitors;
(v) antiangiogenic agents such as those which inhibit the effects of vascular endothelial growth factor, [for example the anti-vascular endothelial cell growth factor antibody bevacizumab (Avastin™); thalidomide; lenalidomide; and for example, a VEGF receptor tyrosine kinase inhibitor such as vandetanib, vatalanib, sunitinib, axitinib and pazopanib;
(vi) gene therapy approaches, including for example approaches to replace aberrant genes such as aberrant p53 or aberrant BRCA1 or BRCA2;
(vii) immunotherapy approaches, including for example antibody therapy such as alemtuzumab, rituximab, ibritumomab tiuxetan (Zevalin®) and ofatumumab; interferons such as interferon a; interleukins such as IL-2 (aldesleukin); interleukin inhibitors for example IRAK4 inhibitors; cancer vaccines including prophylactic and treatment vaccines such as HPV vaccines, for example Gardasil, Cervarix, Oncophage and Sipuleucel-T (Provenge); and toll-like receptor modulators for example TLR-7 or TLR-9 agonists; and
(viii) cytotoxic agents for example fludaribine (fludara), cladribine, pentostatin (Nipent™);
(ix) steroids such as corticosteroids, including glucocorticoids and mineralocorticoids, for example aclometasone, aclometasone dipropionate, aldosterone, amcinonide, beclomethasone, beclomethasone dipropionate, betamethasone, betamethasone dipropionate, betamethasone sodium phosphate, betamethasone valerate, budesonide, clobetasone, clobetasone butyrate, clobetasol propionate, cloprednol, cortisone, cortisone acetate, cortivazol, deoxycortone, desonide, desoximetasone, dexamethasone, dexamethasone sodium phosphate, dexamethasone isonicotinate, difluorocortolone, fluclorolone, flumethasone, flunisolide, fluocinolone, fluocinolone acetonide, fluocinonide, fluocortin butyl, fluorocortisone, fluorocortolone, fluocortolone caproate, fluocortolone pivalate, fluorometholone, fluprednidene, fluprednidene acetate, flurandrenolone, fluticasone, fluticasone propionate, halcinonide, hydrocortisone, hydrocortisone acetate, hydrocortisone butyrate, hydrocortisone aceponate, hydrocortisone buteprate, hydrocortisone valerate, icomethasone, icomethasone enbutate, meprednisone, methylprednisolone, mometasone paramethasone, mometasone furoate monohydrate, prednicarbate, prednisolone, prednisone, tixocortol, tixocortol pivalate, triamcinolone, triamcinolone acetonide, triamcinolone alcohol and their respective pharmaceutically acceptable derivatives. A combination of steroids may be used, for example a combination of two or more steroids mentioned in this paragraph;
(x) targeted therapies, for example Pl3Kd inhibitors, for example idelalisib and perifosine; or compounds that inhibit PD-1, PD-L1 and CAR T.

The one or more other active agents may also be antibiotics (for example anthracyclines like adriamycin, bleomycin, doxorubicin, daunomycin, epirubicin, idarubicin, mitomycin-C, dactinomycin and mithramycin).

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

### Example 1 - Single diastereoisomers of NUC-1031

The (R) and (S) isomers can be separated by HPLC under the following conditions:
Equipment: Agilent 1200™ series with DAD detector
Flow rate: 1.0 mL/min
Column: Chiralpak AD™; 250x4.6 mm ID (normal phase)
Temperature: ambient
Particle size: 20 µm
Feed: dissolved in MeOH; 10g/L
Solvent: n-heptane/IPA 10 ->50% isopropyl alcohol

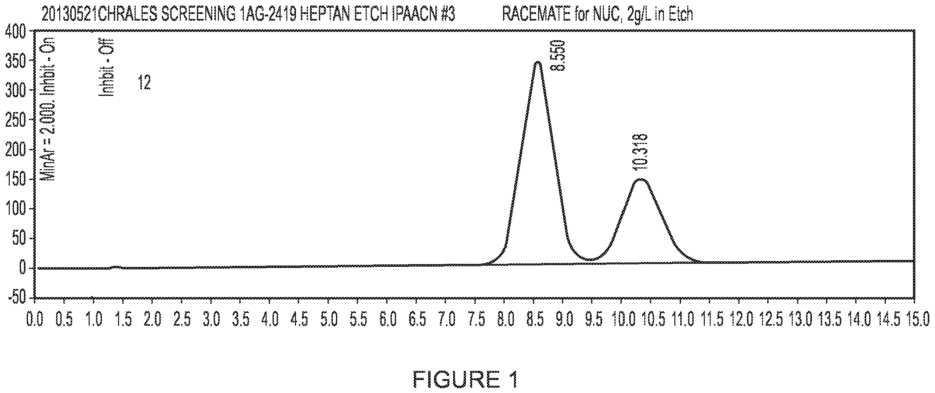

The chromatogram is shown in Figure 1. The (S)-epimer eluted at 8.6 min and the (R)-epimer eluted at 10.3 minutes.

**Characterisation Methods and Materials:** Proton (¹H), carbon (¹³C), phosphorus (³¹P) and fluorine (¹⁹F) NMR spectra were recorded on a Bruker Avance 500 spectrometer at 25°C. Spectra were auto-calibrated to the deuterated solvent peak and all ¹³C NMR and ³¹P NMR were proton-decoupled. The purity of final compounds can be verified by HPLC analysis using Varian Polaris C18-A (10 µM) as an analytic column with a gradient elution of H₂O/MeOH from 100/0 to 0/100 in 35 min. The HPLC analysis was conducted by Varian Prostar (LC Workstation-Varian prostar 335 LC detector).

### 2'-Deoxy-2',2'-difluoro-D-cytidine-5'-O-[phenyl(benzyloxy-L-alaninyl)]-(S)-phosphate 3

(ES+) *m*/*z,* found: (M + Na⁺) 603.14. C₂₅H₂₇F₂N₄O₈NaP required: (M⁺) 580.47.
³¹P NMR (202 MHz, MeOD): δ_{P} 3.66
¹H NMR (500 MHz, MeOD): δ_{H} 7.58 (d, *J* = 7.5 Hz, 1H, *H*-6), 7.38-7.32 (m, 7H, Ar*H*), 7.26-7.20 (m, 3H, Ar*H*), 6.24 (t, *J* = 7.5 Hz, 1H, *H-*1')*,* 5.84 (d, *J* = 7.5 Hz, 1H, *H*-5), 5.20 (AB system, *J*_{AB} = 12.0 Hz, 2H, O*C*H₂Ph), 4.46-4.43 (m, 1H, H-5'), 4.36-4.31 (m, 1H, *H-*5'), 4.25-4.19 (m, 1H, *H*-3'), 4.07 -4.00 (m, 2H, *H*-4', C*H*CH₃), 1.38 (d, *J* = 7.2 Hz, 3H, CHC*H*₃).
¹⁹F NMR (470 MHz, MeOD): δ_{F} - 118.0 (d, *J* = 241 Hz, *F*), - 120.24 (broad d, *J* = 241 Hz, *F).*
¹³C NMR (125 MHz, MeOD): δ_{c} 174.61 (d, ³*J*_{C-p}=5.0 Hz, *C*=O, ester), 167.63 (*C*-NH₂), 157.74 (*C*=O base), 152.10 (d, ²*J*_{C-P} = 7.0 Hz, *C*-Ar), 142.40 (CH-base), 137.22 (*C*-Ar), 130.90, 129.63, 129.39, 129.32, 126.32 (*C*H-Ar), 124.51 (d, ¹*J*_{C-F} = 257 Hz, *C*F₂), 121.47, 121.43 (*C*H-Ar), 96.67 (*C*H-base), 85.92 (broad signal, *C*-1'), 80.31 (*C*-4'), 71.27 (apparent t, ²*J*_{C-F} = 23.7 Hz, *C*-3'), 68.03 (O*C*H₂Ph), 65.73 (d, ²*J*_{C-P}= 5.30 Hz, *C*-5'), 51.66 (*C*HCH₃), 20.42 (d, ³*J*_{C-P} = 6.25 Hz, CH*C*H₃).
Reverse HPLC, eluting with H₂O/MeOH from 100/0 to 0/100 in 35 min, showed one peak of diastereoisomer with *t*_{R} = 22.53 min.

### 2'-deoxy-2',2'-difluoro-D-cytidine-5'-O-[phenyl(benzyloxy- L-alaninyl)]-(R)-phosphate 4.

(ES+) *m*/*z,* found: (M + Na⁺) 603.14. C₂₅H₂₇F₂N₄O₈NaP required: (M⁺) 580.47.
³¹P NMR (202 MHz, MeOD): δ_{P} 3.83
¹H NMR (500 MHz, MeOD): δ_{H} 7.56 (d, *J* = 7.5 Hz, 1H, *H*-6), 7.38-7.31 (m, 7H, Ar*H*), 7.23 - 7.19 (m, 3H, Ar*H*), 6.26 (t, *J* = 7.5 Hz, 1H, *H*-1'), 5.88 (d, *J* = 7.5 Hz, 1H, *H*-5), 5.20 (s, 2H, OC*H*₂Ph), 4.49-4.46 (m, 1H, *H*-5'), 4.38 - 4.34 (m, 1H, *H*-5'), 4.23 - 4.17 (m, 1H, *H*-3'), 4.07 - 4.01 (m, 2H, *H*-4', C*H*CH₃), 1.38 (d, *J* = 7.2 Hz, 3H, CHC*H*₃).
¹⁹F NMR (470 MHz, MeOD): δ_{F} - 118.3 (d, *J* = 241 Hz, *F*), - 120.38 (broad d, *J* = 241 Hz, *F*).
¹³C NMR (125 MHz, MeOD): δ_{C} 174.65 (d, ³*J*_{C-P} = 5.0 Hz, *C*=O, ester), 167.65 (*C*-NH₂), 157.75 (*C*=O base), 152.10 (d, ²*J*_{C-P} = 7.0 Hz, *C*-Ar), 142.28 (*C*H-base), 137.50 (*C*-Ar), 130.86, 129.63, 129.40, 129.32, 126.31 (*C*H-Ar), 124.50 (d, ¹*J*_{C-F} = 257 Hz, *C*F₂), 121.44, 121.40 (*C*H-Ar), 96.67 (*C*H-base), 85.90 (broad signal, *C*-1'), 80.27 (*C*-4'), 71.30 (apparent t, ²*J*_{C-F} = 23.7 Hz, *C*-3'), 68.02 (O*C*H₂Ph), 65.50 (*C*-5'), 51.83 (*C*HCH₃), 20.22 (d, ³*J*_{*C*-P} = 7.5 Hz, CHC*H*₃).
Reverse HPLC, eluting with H₂O/MeOH from 100/0 to 0/100 in 35 min, showed one peak of diastereoisomer with *t*_{R} = 21.87 min.

### Example 2 - NUC-1031 and cisplatin combination study in vitro.

### 2.1 Materials and Methods

### Cell cultures and reagents

A2780, SK-OV-3, OVCAR-3, NCI-H460, NCI-H1975, NCI-H2122, 5637 and HT1376 were cultured in RPMI Medium 1640 (Invitrogen-22400105) supplemented with 10% fetal bovine serum (FBS; Invitrogen-10099141). All the cell lines were maintained in a humidified incubator at 37°C with 5% CO₂. Cell culture media and supplements were purchased from Invitrogen, and tissue culture flasks were purchased from Corning, 96-well plates and 384-well plates were purchased from Greiner. CellTiter-Glo Luminescent Cell Viability Assay kits were purchased from Promega (Promega-G7573), cells counter Vi-Cell was purchased from Beckman, detection instrument Envision was purchased from PerkinElmer.
Paclitaxcel (used as a reference) and cisplatin were purchased from SELLECK, and they were of highest purity available. All compounds attained solubility in DMSO and when diluted into culture media. DMSO, compounds solutions and culture media were warmed to 37°C for the solution preparation and dilutions.

### Cytotoxicity assay

Eight cell lines were allowed to adhere to 96-well plates overnight (100µL/well), for drug treatments with 3.16 fold dilution, 9 dose points, triplicates or vehicle control, compound stock solutions were prepared in DMSO and added to the wells to give the indicated final drug concentrations. Final DMSO concentration was 0.5%. Cellular ATP concentrations were assessed by using the CellTiter-Glo Cell Viability Assay as per the manufacturer's instructions 72 h after drug addition.

### Combination analyses

8 cell lines were allowed to adhere to 384 well plates overnight (60µL/well), for combination study, four combinations of two compounds will be investigated twice, keeping one compound at a fixed concentration while increasing the concentration of the second compound (10 fold dilution, 5 dose points), compound stock solutions were prepared in DMSO and added to the wells to give the indicated final drug concentrations by D300e digital dispenser. Final DMSO concentration was 0.5%.

Cellular ATP concentrations were assessed by using the CellTiter-Glo Cell Viability Assay as per the manufacturer's instructions 72 hours after drug addition.

Thus, the study comprised two stages:

### Stage 1: Single agent IC50 determination

In Stage 1 the IC₅₀ (using 5 or more concentrations) of each individual compound ( cisplatin, gemcitabine and NUC-1031) in the relevant cell lines was determined.

**Table 1: Top concentration of the single agents serial diluted by 3.16-fold in 9 points and tested in triplicates.**

| **Top Concentration (uM)** | | | | |
|---|---|---|---|---|
| **Cell name** | **Cisplatin** | **Gemcitabine** | **NUC-1031** | **Paclitaxcel** |
| A2780 | 198 | 1.98 | 1.98 | 1.98 |
| SK-OV-3 | 198 | 1.98 | 1.98 | 1.98 |
| OVCAR-3 | 198 | 1.98 | 1.98 | 1.98 |
| NCl-H460 | 198 | 1.98 | 1.98 | 1.98 |
| NCl-H1975 | 198 | 1.98 | 1.98 | 1.98 |
| 5673 | 198 | 1.98 | 1.98 | 1.98 |
| HT1376 | 198 | 1.98 | 1.98 | 1.98 |

### Stage 2: Combination treatments

Stage 2 determined the interaction of selected combinations of compounds on cancer cell growth. In total 8 conditions were tested on the relevant cell lines. This means that four combinations of two compounds were investigated twice, keeping one compound at a fixed concentration while increasing the concentration of the second compound.

**Table 2: Combination treatments plan performed in quadruplicates, 5 points with 10-fold dilution.**

| **Tumor type** | **Cell line characteristics** | **Cell line** | **Gemcitabine + cisplatin** | **NUC-1031 + cisplatin** |
|---|---|---|---|---|
| Ovary | Platinum sensitive line | A2780 | X | X |
| Ovary | Moderate sensitivity to Platinum | SK-OV3 | X | X |
| Ovary | Moderate resistance to cisplatin | OVCAR-3 | X | X |
| NSCLC | Platinum sensitive line | NCI-460 | X | X |
| NSCLC | Moderate sensitivity to Platinum | NCI-1975 | X | X |
| Bladder | Sensitive to cisplatin | 5637 | X | X |
| Bladder | Moderate sensitivity to cisplatin | HT-1376 | X | X |

### 2.2 Analytical methods

The following terminology will be utilised to characterise the effect of the compounds combinations:
- "Synergy" as defined by: stronger observed effect of the combined compounds than that predicted from the single compounds effects.
- "Additive" effect as defined by: the observed effect of the combined compounds is equal to that predicted from the sum of the single compounds effects.
- "Antagonism" as defined by: significantly weaker effect of the combined compounds than predicted from the single compounds effects.

### Chou-Talalay Method

The Chou-Talalay method for drug combination is based on the median-effect equation, derived from the mass-action law principle, the resulting combination index (CI) theorem of Chou-Talalay offers quantitative definition for additive effect (CI = 1), synergism (Cl< 1) in drug combinations.

### Bliss independence model

The method compares the observed combination response (YO) with the predicted combination response (YP), which was obtained based on the assumption that there is no effect from drug-drug interactions.

Suppose two drugs, A and B, both inhibit tumor growth: drug A at dose a inhibits Y^{a} percent of tumor growth and drug B at dose b inhibits Y^{b} percent of tumor growth. If two drugs work independently, the combined percentage inhibition
Y^{ab}, P can be predicted using the complete additivity of probability theory as
Y^{ab}, P = Y^{a} +Y^{b}-Y^{a}Y^{b}

### Curve shift analysis

Suppose two drugs work independently, keep drug A at a fixed concentration and vary drug B's concentration normalize the combination effect based on fixed A's concentration, compares the dose effect curves obtained from drug B, a leftward shift of combination dose-effect curves relative to synergy, a rightward shift indicates antagonism, and overlapping indicate additive.

### 2.3 Results

### Stage 1: cytotoxicity assay with single agents

In Stage 1 of the study the cytotoxicity of the single agents cisplatin, NUC-1031 and gemcitabine have been investigated in order to inform the most appropriate concentrations for the combination work in Stage 2.

**Table 3: Summary of absolute IC₅₀, relative IC₅₀ and maximum inhibition results for the single agents treatment in the relevant cancer cell lines tested.**

| No. | Cell Line | CTG Assay | | | | | | | | |
|---|---|---|---|---|---|---|---|---|---|---|
| | | Cisplatin | | | Gemcitabine | | | NUC-1032 | | |
| | | Ab IC50(uM) | IC50(uM | Max Inhibition_% | Ab IC50(uM) | IC50(uM | Max Inbibition_% | Ab IC50(uM) | IC50(uM) | Max Inhibition_% |
| 1 | A2780 | 10.96 | 10.07 | 99.05 | 0.01 | 0.01 | 81.15 | 0.025 | 0.016 | 79.15 |
| 2 | SK-OV3 | 45.61 | 33.73 | 86.42 | 0.02 | 0.02 | 65.74 | 0.07 | 0.06 | 61.59 |
| 3 | OVCAR-3 | 28.32 | 23.46 | 79.59 | >1.98 | 0.02 | 6.95 | >1.98 | 0.20 | 11.76 |
| 4 | NCl-H460 | 2.59 | 2.57 | 97.56 | 0.01 | 0.01 | 96.28 | 0.04 | 0.04 | 91.98 |
| 5 | NCl-H1975 | 69.55 | 69.23 | 103.60 | 0.08 | 0.02 | 62.12 | >1.98 | 0.37 | 37.91 |
| 6 | 5637 | 13.70 | 13.21 | 101.74 | 0.01 | 0.01 | 84.49 | 0.20 | 0.12 | 77.88 |
| 7 | HT1376 | 20.51 | 18.36 | 71.90 | >1.98 | >1.98 | 9.60 | >1.98 | >1.98 | -2.54 |

### Data overview - Summary of results from all three analytical methods

Table 4 below shows the outcome of the analysis utilising the 3 methodologies (Chou-Talalay, Bliss Independence and Curve Shift) to characterise the effect of the combined compounds NUC-1031 and cisplatin.

**Table 4: Outcome of the 3 analytical methodologies utilised to assess combined compounds effect on cancer cells growth**

| Cell line | Fixed Conc. | Methodology | | |
|---|---|---|---|---|
| | | Chou-Talalay | Bliss independance | Curve shift |
| A2780 (Ovary) | Acelarin 0,025uM | Additive | Additive | Additive |
| | Cisplatin 11uM | | | |
| Sk-OV3 (Ovary) | Acelarin 0.072uM | Unmeasurable | Additive | Additive |
| | Cisplatin 45.6uM | | | |
| OVCAR-3 (Ovary) | Acelarin 1.98uM | Unmeasurable | Additive | Additive |
| | Cisplatin 28.3uM | | | |
| NCl-H460 (Lung) | Acelarin 0.04uM | Antagonism | Antagonism | Additive |
| | Cisplatin 2.6uM | | Cisplatin 2.6uM+Acelarin 0.0198uM | |
| NCl-H1975 (Lung) | Acelarin 1.98uM | Additive | Additive | Additive |
| | Cisplatin 70uM | | | |
| 5637(Bladder) | Acelarin 0.199uM | Synergy | Additive | Antagonism |
| | Cisplatin 13.7uM | | | |
| HT-1376(Bladder) | Acelarin 1.98uM | Synergy | Synergy | Synergy |
| | Cisplatin 20.51uM | | | |

The concordance of the results between all three analytical methods showed that synergy was observed with two compound combinations against the HT1376 cancer cell line and this has been summarised in table 5.

**Table 5: Synergy of combined treatments observed across the three methods**

| Cell line | Fixed concentration | Serial dose-effect |
|---|---|---|
| | Gemcitabine | |
| HT1376 (Bladder) | 1.98µM) | Cisplatin |
| | NUC-1031 | |
| HT1376 (Bladder) | 1.98µM | Cisplatin |

### Individual methodology results

### • Data analysed using the Chou-Talalay method

### CI < 1, suggesting synergy

| CI Data for Non-Constant Combo: GemCis (Cis+Gem) | | | | |
|---|---|---|---|---|
| **Dose Cis** | **Dose Gem** | **Effect** | **CI** | **Cell Line** |
| 198.0 | 0.007 | 0.8952 | 0.518 | A2780 |
| 11.0 | 1.98 | 0.8525 | 0.528 | A2780 |
| 11.0 | 0.198 | 0.7973 | 0.489 | A2780 |
| 198.0 | 0.008 | 0.931 | 0.341 | NCI-H460 |
| 19.8 | 0.008 | 0.7922 | 0.375 | NCI-H460 |
| 198.0 | 0.08 | 0.7688 | 0.15028 | NCI-H1975 |
| 19.8 | 0.08 | 0.6851 | 0.17708 | NCI-H1975 |
| 1.98 | 0.08 | 0.6976 | 0.149 | NCI-H1975 |
| 70.0 | 0.198 | 0.7231 | 0.33835 | NCI-H1975 |
| 70.0 | 0.0198 | 0.4228 | 0.65284 | NCI-H1975 |
| 19.8 | 0.012 | 0.7909 | 0.68758 | 5637 |
| 1.98 | 0.012 | 0.7836 | 0.20073 | 5637 |
| 0.198 | 0.012 | 0.7522 | 0.19804 | 5637 |
| 0.0198 | 0.012 | 0.7314 | 0.22947 | 5637 |
| 13.7 | 0.198 | 0.9446 | 0.31724 | 5637 |
| 13.7 | 0.0198 | 0.8726 | 0.33306 | 5637 |
| 198.0 | 1.98 | 0.7601 | 0.46232 | HT1376 |
| 19.8 | 1.98 | 0.6007 | 0.59809 | HT1376 |
| 20.51 | 1.98 | 0.6572 | 0.4459 | HT1376 |
| 20.51 | 0.198 | 0.5269 | 0.3652 | HT1376 |

| CI Data for Non-Constant Combo: AceCis (Cis+Ace) | | | | |
|---|---|---|---|---|
| **Dose Cis** | **Dose Ace** | **Effect** | **CI** | **Cell Line** |
| 198.0 | 0.025 | 0.9004 | 0.505 | A2780 |
| 11.0 | 0.198 | 0.6605 | 0.642 | A2780 |
| 2.6 | 0.198 | 0.7749 | 0.595 | NCI-H460 |
| 198.0 | 1.98 | 0.8097 | 0.27205 | NCI-H1975 |
| 70.0 | 0.198 | 0.5335 | 0.65375 | NCI-H1975 |
| 198.0 | 0.199 | 0.9794 | 0.57158 | 5637 |
| 1.98 | 0.199 | 0.8218 | 0.60206 | 5637 |
| 0.198 | 0.199 | 0.7971 | 0.69873 | 5637 |
| 0.0198 | 0.199 | 0.8019 | 0.66363 | 5637 |
| 13.7 | 0.198 | 0.8715 | 0.56581 | 5637 |
| 19.8 | 1.98 | 0.4966 | 0.27588 | HT1376 |
| 20.51 | 1.98 | 0.6016 | 0.22343 | HT1376 |
| 20.51 | 0.198 | 0.3899 | 0.36726 | HT1376 |
| 20.51 | 0.0198 | 0.274 | 0.49796 | HT1376 |
| 20.51 | 0.00198 | 0.2662 | 0.50941 | HT1376 |
| 20.51 | 1.98E-4 | 0.3175 | 0.44121 | HT1376 |

### Cl > 1, suggesting antagonism

| CI Data for Non-Constant Combo: GemCis (Cis+Gem) | | | | |
|---|---|---|---|---|
| **Dose Cis** | **Dose Gem** | **Effect** | **CI** | **Cell Line** |
| 19.8 | 0.007 | 0.519 | 18.3616 | A2780 |
| 1.98 | 0.007 | 0.3904 | 302.897 | A2780 |

| CI Data for Non-Constant Combo: AceCis (Cis+Ace) | | | | |
|---|---|---|---|---|
| **Dose Cis** | **Dose Ace** | **Effect** | **CI** | **Cell Line** |
| 11.0 | 0.00198 | 0.3049 | 2.22423 | A2780 |
| 1.98 | 0.04 | 0.3557 | 2.266 | NCI-H460 |
| 0.198 | 0.04 | 0.2166 | 4.306 | NCI-H460 |
| 2.6 | 0.0198 | 0.1942 | 6.145 | NCI-H460 |
| 2.6 | 0.00198 | 0.2357 | 2.665 | NCI-H460 |

### • Data analysed using the Curve shift method

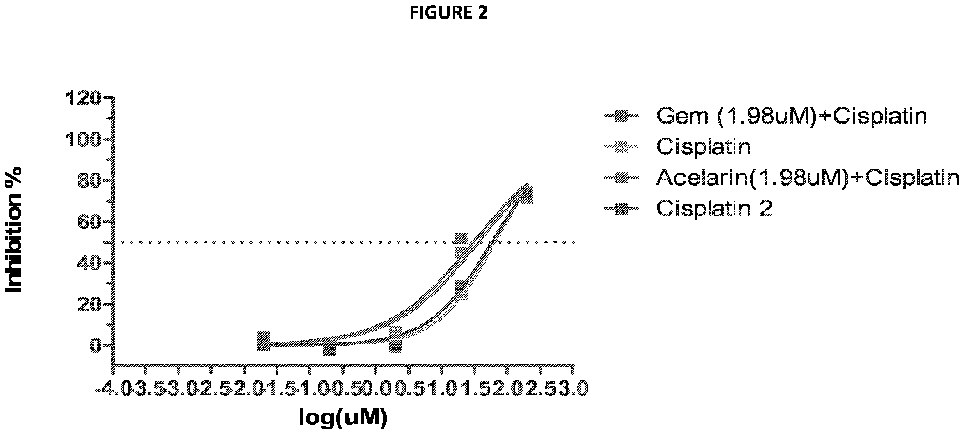

Synergy effect shown below in the bladder cancer cell line HT1376 is shown in figure 2

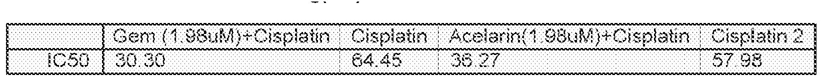

### • Data analysed using the Bliss Independence method

| Cell line | Fixed concentration | Serial dose-effect |
|---|---|---|
| **Synergy** | | |
| | Gemcitabine | |
| HT1376 | 1.98uM | Cisplatin |
| | Cisplatin | |
| HT1376 | 20.51 uM | Gemcitabine |
| | NUC-1031 | |
| HT1376 | 1.98uM | Cisplatin |
| | Cisplatin | |
| HT1376 | 20.51uM | NUC-1031 |

### EXAMPLE 3 - Further NUC-1031 and cisplatin combination in vitro study

### Cell Culture and Reagents

SKOV3 cells were purchased from American Type Culture Collection (ATCC). They were cultured at 37°C and 5% CO₂ in Gibco™ RMPI Medium 1640 + GlutaMAX™-I (Life Technologies; 61870-010) with 10% Gibco™ FBS (Life Technologies; 10270-106) and 1% Gibco™ Penicillin-Streptomycin (Life Technologies; 15140122), known as complete medium, in a cell culture flask. Cisplatin was purchased from TEVA UK Limited (PL 00289/1146). Fluvastatin and Sulforaphane were both purchased from Merck Millipore Corporation (Catalogue numbers 344096 and 574215 respectively). (S)-NUC-1031 was provided by NuCana® Ltd.

### Cell Culture with Drug Applications

SKOV3 cells were plated at 250 cells in 200µl per well in in the middle 60 wells of Costar® 3596 96-well plates, with the outer 36 wells filled with 200µl PBS each. The cells were allowed to grow in complete medium for 48 hours before different drug compounds were added in. For single agent, cisplatin, (S)-NUC-1031, fluvastatin and sulforaphane in complete medium at 10 different concentrations were added into the middle 60 wells (Day 0). Each concentration was loaded in sextuplicate. Cells were exposed to the compounds for 24 hours and the compounds were replaced by pure complete medium. The experiment with fluvastatin was repeated with 4-day exposure instead of 24 hours. The plates were fixed with 50µl 25% TCA solution at 4°C for 60 minutes on day 4 after the drug compounds were added for Sulforhodamine B assay (see below).

For combination of two agents, different concentrations of cisplatin and fluvastatin, and two fixed concentrations of cisplatin combined with various concentrations of fluvastatin were plated in a Costar® 3596 96-well plate in triplicate in the middle 60 wells. The outer 36 wells were filled with 200µl PBS each (Day 0). Cells were allowed 24-hour exposure to the drug compounds, and the compounds were aspirated and replaced with pure complete medium on day 1. The experiment was repeated with cisplatin with sulforaphane, and cisplatin with (*S*)-NUC-1031, and each concentration was loaded in triplicate as well. The plates were fixed with 50µl 25% TCA solution at 4°C for 60 minutes on day 4 for Sulforhodamine B assay (see below).

### Sulforhodamine B (SRB) colorimetric assay

After adding the TCA solution, the plates were washed under running tap water 10 times and were allowed to dry in the oven at 50°C. The cells were then stained with 50µl SRB dye, and the dye was washed off with 1% glacial acetic acid for 4 times 30 minutes after SRB addition. The plates were then dried in the oven. SRB dye was allowed to dissolve in 150µl 10mM Tris buffer solution for 60 minutes on a rocker. The plates were read on Biohit BP800 Microplate Reader (Biohit Healthcare) and the absorbance was measured at 540nm. The results of both single agent and combination experiments were used to calculate IC₅₀ of the drug compounds using Prism Software (GraphPad), which is the concentration of drug that causes 50% inhibition. As for the results from combination of two agents, they were used to calculate combination index (CI) of the compounds using CalcuSyn Software (Biosoft).

### RESULTS:

IC50 of cisplatin and sulforaphane were 2.436µM and 7.002µM respectively. IC50, of fluvastatin and NUC-1031 could not be calculated due to unobservable inhibitory effects. CI values of cisplatin with fluvastatin ranged from 0.355 to 0.557, showing synergy. CI values of cisplatin with sulforaphane ranged from 0.891 to 1.474, showing antagonism. CI values of cisplatin with NUC-1031 at 0.1 - 0.5µM and 1 - 2µM on SKOV3 cells ranges from 0.871 to 0.957, and 1.067 to 1.756 respectively.

### Conclusion

Synergistic inhibition was found at low concentrations of NUC-1031 (0.1 - 0.5µM) with cisplatin on SKOV3 cells, which makes it more likely to be used clinically as lower concentrations of drugs usually give lower toxicity. This result can act as the basis for further investigation into the precise optimal combination dose before testing in patients to ensure efficacy and safety. Personalised treatment by evaluating the cytotoxicity and side effects of the combination in individuals would be optimal due to diversities of genetic profile and drug response, such that each patient can receive drugs at optimal doses.

### EXAMPLE 4 - Pharmacokinetic analysis of dFdCTP concentrations from the ABC-008 clinical study (NUC-1031 in combination with cisplatin) and comparison with results from the ProGem1 clinical study (NUC-1031 alone).

Initial pharmacokinetic analysis was conducted on samples obtained from the first three patients on the ABC-008 clinical study.

The patient details are as follows:
Patient 1: 71 years old - Metastatic Biliary Tract Carcinoma - Starting dose: 625mg/m² (S)-NUC-1031 + 25mg/m² cisplatin
Patient 2: 78 years old - Metastatic Biliary Tract Carcinoma - Starting dose: 625mg/m² (S)-NUC-1031 + 25mg/m² cisplatin
Patient 3: 75 years old - Metastatic Biliary Tract Carcinoma - Starting dose: 625mg/m² (S)-NUC-1031 + 25mg/m² cisplatin

Both NUC-1031 and cisplatin were administered on days 1 and 8 of a 21 day cycle.

An appropriate dosage of 625mg/m² of NUC-1031 was prepared in a Luerlock syringe. The dosage given was based on the subject's height and weight using a standard body surface area (BSA) calculation. A polyethylene extension line was primed with up to 1.5 ml of Flushing Solution, prior to connecting the syringe containing NUC-1031 to the extension line.

The extension line was connected to the patient's Central Venous Access Device (CVAD), and NUC-1031 was injected at a rate of 20 ml/hour using a syringe pump.
Once the injection was complete the NUC-1031 syringe was disconnected from the extension line, and the extension line was then flushed with an additional volume of up to 3 ml of Flushing solution.

### Materials and methods

### 1. Materials

dFdCTP reference compound was obtained from Biorbyt, UK. Lymphoprep from STEMCELL Technologies Inc., UK. Perchloric acid (PCA), ammonium acetate (NH4Ac) and ammonia were all obtained from Sigma Aldrich, UK. LC-MS grade Water, methanol, acetonitrile and formic acid were all obtained from Fisher Scientific, UK.

### 2. Methods

*A. Blood collection and PBMCs preparation:* 6 ml of blood was collected using heparinised blood collection tubes. After centrifugation and separation of plasma, buffycoat was collected and transferred to new test tube containing 3ml of Lymphoprep density gradient. After centrifugation, the upper interface containing the PBMC layer was transferred to new test tube. After washing with phosphate buffered saline (PBS), PBMCs were resuspended in 100 µl PBS. Then, another 100 µl of 0.8 M PCA was added and the mixture was vortex mixed and centrifuged followed by transfer of 100 µl supernatant to new test tube. The PCA extracts were stored at -80°C until time of analysis.
*B. Sample extraction (PBMCs):* PCA extracts were buffered using 50 µl of 1M NH₄Ac, then neutralised using 20 µl of 10% ammonia solution. Finally, 5µl containing the internal standard 8-ChloroATP and 5 µl deionised water were then added. The extracts were transferred to LC-MS vials and 10 µl were injected into the UPLC-MS/MS system.

### 3. Chromatography method and sample analysis

10mg/mL stock solution of the analyte was prepared and aliquot frozen at -80°C until use. The analyte was resolved using an ultra-performance liquid chromatography system (Accela UPLC, Thermo Scientific, UK) equipped with a Biobasic AX, 5 µm, 50 × 2.1 mm column (Thermo Electron Corporation, Murrieta, CA, USA) and a mobile phase consisting of a mixture of 10 mM NH₄Ac in ACN/H₂O (30 : 70 v/v), pH 6.0 (A), and 1 mM NH₄Ac in ACN/H₂O (30 : 70 v/v), pH 10.5 (B). The mobile phase gradient was employed, comprising: buffer A = 95% at 0 - 0.5 min, from 95 to 0% over 1.25 minutes, held at 0% for 1.75 minute, from 0 to 95% over 0.1 minutes, ending with 95% for 2.9 minutes, all at a flow rate of 500 µl/min.

### 4. Mass Spectrometry method

Eluting compounds of interest were detected using a triple stage quadrupole Vantage mass spectrometry system (Thermo Scientific, UK) equipped with an electrospray ion source. Samples were analyzed in the Multiple Reaction Monitoring (MRM), positive (+ve) and negative (-ve) ion modes at a spray voltage of 3500 and 3000 V, respectively. Nitrogen was used as sheath and auxiliary gases at a flow rate of 50 and 20 arbitrary units, respectively. Argon was used as collision gas with pressure of 1.5 mTorr.

### Results

### Initial results are presented in Tables 9 and 10

**Table 10: Mean PK parameters comparison of ProGem1 and ABC-008 studies**

| Mean plasma PK Parameters (no normalisation) for NUC-1031 | | |
|---|---|---|
| | ProGem1 (n=67) (NUC-1031 as a single agent) | ABC-008 (n=3) (NUC-1031 in combination with cisplatin) |
| Mean Cmax (µg/ml) | 66.5 | 124.6 |
| Median Tₘₐₓ (hr) | 0.5 | 0.5 |
| Mean AUC₀₋₂₄ (µg/ml.hr) | 66.5 | 142.8 |
| Mean terminal t_{1/2}(hr) | 0.9 | 0.5 |
| Mean clearance (L/hr) | 5.2 | 8.3 |

**Table 10: Mean PK parameters comparison of ProGem1 and ABC-008 studies**

| Mean Intracellular PK Parameters (no normalisation) for dFdCTP | | |
|---|---|---|
| | ProGem1 (n=67) (NUC-1031 as a single agent) | ABC-008 (n=3) (NUC-1031 in combination with cisplatin) |
| Mean Cₘₐₓ (pmol/million cells) | 391.8 | 350.4 |
| Median Tₘₐₓ (hr) | 0.5 | 1 |
| Mean AUC₀₋₂₄ (pmol/million cells.hr) | 1379.0 | 1130.0 |
| Mean terminal t_{1/2}(hr) | 5.7 | 20.6 |
| Mean clearance (L/hr) | 0.2 | 0.3 |

### Discussion

NUC-1031 plasma PK parameters showed a 2.1 fold AUC increase and a 1.9 fold increase in Cmax compared to ProGem1 (the First-In-Human Phase I study with single agent NUc-1031). The NUC-1031 plasma PK parameters also showed a 3.6 fold increase in half-life compared to single agent NUC-1031.

Intracellular dFdCTP (the active anti-cancer moiety) parameters were very similar to ProGem1 with the notable exception of longer t1/2. This longer t1/2 may be due to maintained higher levels of intracellular dFdCTP over the 4 hours period of PK sampling. The synergy observed in the dFdCTP levels following NUC-1031 with cisplatin treatment has significant potential clinical implications, including broader clinical utility to treat cancers where high dFdCTP levels are required over a longer time period to block tumour growth and in treating recurrent cancers following single agent use.

It will be appreciated that the increased half-life of dFdCTP observed on treatment using the combinations of gemcitabine-[phenyl-benzoxyl-_{L}-alaninyl]-phosphate, or pharmaceutically acceptable salt or solvate thereof, with platimum-based anticancer agents described herein, provides advantages in the treatment of cancer in a number of contexts. A major advantage is found in the dosing flexibility that this increased half-life confers. For example, such medical uses allow effective treatment regimens in which incidences of treatment using the active agents are less frequent than those currently employed. Merely by way of example, the treatments of the invention can be provided to a patient in a single daily incidence of treatment, rather than requiring multiple administrations over the course of day. Suitably incidences of treatment, for example such single incidences of treatment, may require only relatively rapid provision of the active agents, rather than prolonged administration, for example by way of infusion. The gemcitabine-[phenyl-benzoxyl-_{L}-alaninyl]-phosphate and platinum-based anticancer agent with which it is to be used may be formulated (either in combination or individually) as a medicament for single daily administration to a patient. Medicaments of this sort, for single daily administration, may be useful in the treatment of recurrent cancers.

Treatments in accordance with the invention, using gemcitabine-[phenyl-benzoxyl-_{L}-alaninyl]-phosphate in combination with a platinum-based anticancer agent, whether in combination or sequentially, may be used in incidences of treatment provided every two days, every, three days, every four days, every five days, every six days, or every week. Indeed, the treatments in accordance with the invention may be used in incidences of treatment provided one, two, or three weeks apart from one another.

### EXAMPLE 5 - Comparison of key progression free survival time points obtained to date in the ABC-08 study and associated radiological responses, versus median time points established for progression free survival in the ABC-02 study in single agent Gemcitabine and Gemcitabine/Cisplatin combination therapy.

### ABC-02 Background:

The ABC-02 study established Gemcitabine/Cisplatin combination as the superior standard of care treatment in comparison to Gemcitabine alone in the metastatic biliary setting. The established median for progression free survival for patients receiving Gemcitabine/Cisplatin was 8 months. The established median for progression free survival for patients receiving Gemcitabine as single agent therapy was 5 months.
(Valle J, Wasan H, Palmer DH et al in 2010)

### ABC-08 Comparison:

Progression free survival time points obtained to date on the ABC-08 study have exceeded the established median for both single agent Gemcitabine and Gemcitabine/Cisplatin combination therapy. Specific instances are cited below:
Patient 02: - had a 60% reduction in NUC-1031 dosage to 375mg/m² and an accompanying 25% reduction in cisplatin. Nevertheless, as detailed below she proceeded to exhibit a series of sustained reductions in tumour volume.

This patient has achieved a progression free survival time point of 9 months and is ongoing. This is the longest observed progression free survival time point on the ABC-08 study and currently exceeds the median established by the ABC-02 study.

This same patient has demonstrated sustained ongoing reductions in tumour volume across multiple radiological assessments.
- Month 3 Scan: 17% reduction - Stable Disease
- Month 6 Scan: 24% reduction - Stable Disease
- Month 9 Scan: 41% reduction - Partial response.

Patient 05 - 55 years old - Metastatic Biliary Tract Carcinoma - Starting dose: 625mg/m² (S)-NUC-1031 + 25mg/m² cisplatin

This patient has achieved a progression free survival time point of 5.5 months and is currently ongoing, surpassing the median progression free survival time point of 5 months established for patients receiving single agent Gemcitabine.

This same patient has demonstrated a significant reduction in tumour volume in the first radiological assessment:
- Month 3 Scan: 54% reduction - Partial response.

Although these results are from individual patients they represent promising clinical results for the combination of the invention.

### Clauses

1. Gemcitabine-[phenyl-benzoxy-L-alaninyl)]-phosphate, or a pharmaceutically acceptable salt or solvate thereof for use in treating cancer in combination with platinum-based anticancer agent selected from cisplatin, picoplatin, lipoplatin and triplatin.
2. Gemcitabine-[phenyl-benzoxy-L-alaninyl)]-phosphate for use of clause 1 wherein the platinum-based anticancer agent is cisplatin.
3. Gemcitabine-[phenyl-benzoxy-L-alaninyl)]-phosphate for use of clause 1 or clause 2, wherein the gemcitabine-[phenyl-benzoxy-L-alaninyl)]-phosphate is gemcitabine-[phenyl-benzoxy-L-alaninyl)]-(S)-phosphate in substantially diastereomerically pure form.
4. Gemcitabine-[phenyl-benzoxy-L-alaninyl)]-phosphate for use of clause 1 or clause 2, wherein the gemcitabine-[phenyl-benzoxy-L-alaninyl)]-phosphate is a mixture of phosphate diastereoisomers.
5. Gemcitabine-[phenyl-benzoxy-L-alaninyl)]-phosphate for use of any one of clauses 1 to 4, wherein the gemcitabine-[phenyl-benzoxy-L-alaninyl)]-phosphate is in the form of the free base.
6. Gemcitabine-[phenyl-benzoxy-L-alaninyl)]-phosphate for use of any one of clauses 1 to 5, wherein the gemcitabine-[phenyl-benzoxy-L-alaninyl)]-phosphate is administered intravenously.
7. Gemcitabine-[phenyl-benzoxy-L-alaninyl)]-phosphate for use of any one of clauses 1 to 6, wherein the cancer is a solid tumour, e.g. a cancer selected from ovarian cancer, bladder cancer, and biliary tract cancer.
8. Gemcitabine-[phenyl-benzoxy-L-alaninyl)]-phosphate for use of clause 7, wherein the cancer is biliary tract cancer, e.g. a cancer selected from gallbladder cancer, distal bile duct cancer, ampullary cancer, hilar cholangiocarcinoma and intra-hepatic cholangiocarcinoma.
9. Gemcitabine-[phenyl-benzoxy-L-alaninyl)]-phosphate for use of any one of clauses 1 to 8, wherein the cancer is relapsed.
10. Gemcitabine-[phenyl-benzoxy-L-alaninyl)]-phosphate for use of any one of clauses 1 to 8, wherein the cancer is metastatic.
11. Gemcitabine-[phenyl-benzoxy-L-alaninyl)]-phosphate for use of any one of clauses 1 to 10, wherein the cancer is refractory, resistant or partially resistant to the platinum-based anticancer agent.
12. Gemcitabine-[phenyl-benzoxy-L-alaninyl)]-phosphate for use of any one of clauses 1 to 9, wherein the cancer is sensitive to the platinum-based anticancer agent.
13. Gemcitabine-[phenyl-benzoxy-L-alaninyl)]-phosphate for use of any one of clauses 1 to 12, wherein the dose of NUC-1031 administered at each administration event is between 250 mg/m² and 1250 mg/m² and the dose of the platinum-based anticancer agent administered at each administration event is between 10 mg/m² and 200 mg/m².
14. A method of treating cancer, the method comprising administering to a subject in need thereof a therapeutically effective amount of gemcitabine-[phenyl-benzoxy-L-alaninyl)]-phosphate, or a pharmaceutically acceptable salt or solvate thereof, in combination with a platinum-based anticancer agent selected from cisplatin, picoplatin, lipoplatin and triplatin.
15. A method of clause 14, wherein the platinum-based anticancer agent is cisplatin.
16. A method of clause 14 or clause 15, wherein the gemcitabine-[phenyl-benzoxy-L-alaninyl)]-phosphate is gemcitabine-[phenyl-benzoxy-L-alaninyl)]-(S)-phosphate in substantially diastereomerically pure form.
17. A method of clause 14 or clause 15, wherein the gemcitabine-[phenyl-benzoxy-L-alaninyl)]-phosphate is a mixture of phosphate diastereoisomers.
18. A method of any one of clauses 14 to 17, wherein the gemcitabine-[phenyl-benzoxy-L-alaninyl)]-phosphate is in the form of the free base.
19. A method of any one of clauses 14 to 18, wherein the gemcitabine-[phenyl-benzoxy-L-alaninyl)]-phosphate is administered intravenously.
20. A method of any one of clauses 14 to 19, wherein the cancer is a solid tumour, e.g. a cancer selected from ovarian cancer, bladder cancer, and biliary tract cancer.
21. A method of clause 20, wherein the cancer is biliary tract cancer, e.g. a cancer selected from gallbladder cancer, distal bile duct cancer, ampullary cancer, hilar cholangiocarcinoma and intra-hepatic cholangiocarcinoma.
22. A method of any one of clauses 14 to 21, wherein the cancer is relapsed.
23. A method of any one of clauses 14 to 22, wherein the cancer is metastatic.
24. A method of any one of clauses 14 to 23, wherein the cancer is refractory, resistant or partially resistant to the platinum-based anticancer agent.
25. A method of any one of clauses 14 to 23, wherein the cancer is sensitive to the platinum-based anticancer agent.
26. A method of any one of clauses 14 to 25, wherein the dose of NUC-1031 administered at each administration event is preferably between 250 mg/m² and 1250 mg/m² and the dose of the platinum-based anticancer agent administered at each administration event is between 10 mg/m² and 200 mg/m².
27. A method of any one of clauses 14 to 26, wherein administration of the combination provides an intra-cellular t_{1/2} of dFdCTP of more than 10 hours.
28. A method of any one of clauses 14 to 27, wherein administration of the combination provides an intra-cellular t_{1/2} of dFdCTP of more than 18 hours.
29. A pharmaceutical formulation comprising gemcitabine-[phenyl-benzoxy-L-alaninyl)]-phosphate, or a pharmaceutically acceptable salt or solvate thereof, together with a platinum-based anticancer agent selected from cisplatin, picoplatin, lipoplatin and triplatin, and at least one pharmaceutically acceptable excipient.
30. A kit comprising two separate formulations to be used together, the formulations being:
   a first formulation comprising gemcitabine-[phenyl-benzoxy-L-alaninyl)]-phosphate, or a pharmaceutically acceptable salt or solvate thereof, and at least one pharmaceutically acceptable excipient; and
   a second formulation comprising a platinum-based anticancer agent selected from cisplatin, picoplatin, lipoplatin and triplatin and at least one pharmaceutically acceptable excipient.

## Claims

1. A formulation comprising gemcitabine-[phenyl-benzoxy-L-alaninyl)]-phosphate, or a pharmaceutically acceptable salt or solvate thereof and at least one pharmaceutically acceptable excipient for use in treating cancer in combination with a platinum-based anticancer agent selected from cisplatin, picoplatin, lipoplatin and triplatin.

2. The formulation for use of claim 1, wherein the platinum-based anticancer agent is cisplatin.

3. The formulation for use of claim 1 or claim 2, wherein the gemcitabine-[phenyl-benzoxy-L-alaninyl)]-phosphate is gemcitabine-[phenyl-benzoxy-L-alaninyl)]-(S)-phosphate in substantially diastereomerically pure form.

4. The formulation for use of claim 1 or claim 2, wherein the gemcitabine-[phenyl-benzoxy-L-alaninyl)]-phosphate is a mixture of phosphate diastereoisomers.

5. The formulation for use of any one of claims 1 to 4, wherein the gemcitabine-[phenyl-benzoxy-L-alaninyl)]-phosphate is in the form of the free base.

6. The formulation for use of any one of claims 1 to 5, wherein the gemcitabine-[phenyl-benzoxy-L-alaninyl)]-phosphate is administered intravenously.

7. The formulation for use of any one of claims 1 to 6, wherein the cancer is a solid tumour, e.g. a cancer selected from ovarian cancer, bladder cancer, and biliary tract cancer.

8. The formulation for use of claim 7, wherein the cancer is biliary tract cancer, e.g. a cancer selected from gallbladder cancer, distal bile duct cancer, ampullary cancer, hilar cholangiocarcinoma and intra-hepatic cholangiocarcinoma.

9. The formulation for use of any one of claims 1 to 8, wherein the cancer is relapsed.

10. The formulation for use of any one of claims 1 to 8, wherein the cancer is metastatic.

11. The formulation for use of any one of claims 1 to 10, wherein the cancer is refractory, resistant or partially resistant to the platinum-based anticancer agent.

12. The formulation for use of any one of claims 1 to 9, wherein the cancer is sensitive to the platinum-based anticancer agent.

13. The formulation for use of any one of claims 1 to 12, wherein the dose of gemcitabine-[phenyl-benzoxy-L-alaninyl)]-phosphate administered at each administration event is between 250 mg/m² and 1250 mg/m² and the dose of the platinum-based anticancer agent administered at each administration event is between 10 mg/m² and 200 mg/m².

14. The formulation for use of any one of claims 1 to 12, adapted for parenteral administration.

15. The formulation for use of any one of claims 1 to 12, adapted for oral administration.
